(19) Europäisches Patentamt
European Patent Office
Office européen des brevets

(11) **EP 4 715 736 A1**

(12) **EUROPEAN PATENT APPLICATION**

(43) Date of publication:
**25.03.2026 Bulletin 2026/13**

(21) Application number: **25199617.9**

(22) Date of filing: **02.09.2025**

(51) International Patent Classification (IPC):
**G06T 7/00** (2017.01)   **G06T 7/33** (2017.01)

(52) Cooperative Patent Classification (CPC):
**G06T 7/33; G06T 7/0016**; G06T 2207/30096;
G06T 2207/30184

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **24.09.2024 JP 2024164936**

(71) Applicant: **Casio Computer Co., Ltd.
Tokyo 151-8543 (JP)**

(72) Inventor: **Saito, Atsushi
Hamura-shi, Tokyo, 205-8555 (JP)**

(74) Representative: **Grünecker Patent- und
Rechtsanwälte
PartG mbB
Leopoldstraße 4
80802 München (DE)**

(54) **POINT MATCHING IN IMAGES**

(57)     A processor (100) acquires a first image and a second image each including a plurality of partial image areas, performs matching between a plurality of partial image areas included in the first image and a plurality of partial image areas included in the second image, and accepts correction by a user on matching between a partial image area in the first image and a partial image area in the second image. The processor (100), using a matching associated with correction by the user as a constraint condition, performs matching between a plurality of partial image areas other than the partial image area associated with the constraint condition in the first image and a plurality of partial image areas other than the partial image area associated with the constraint condition in the second image again.

FIG. 9

```
        ┌────────────────┐
        │     IMAGE      │
        │  PROCESSING    │
        └───────┬────────┘
                │
   ┌────────────────────────────┐
   │ ACQUIRE FIRST CAPTURED IMAGE │──── S101
   │ AND SECOND CAPTURED IMAGE   │
   └────────────┬───────────────┘
                │
        ┌────────────────┐
        │    DETECT       │──── S102
        │ OBJECT OF       │
        │ INTEREST        │
        └───────┬────────┘
                │
        ┌────────────────┐
        │    SELECT       │──── S103
        │ OBJECT OF       │
        │ INTEREST        │
        └───────┬────────┘
                │◄──────────────────────┐
        ┌────────────────┐              │
        │    MATCH        │──── S104     │
        │ OBJECTS OF      │              │
        │ INTEREST        │              │
        └───────┬────────┘              │
                │                        │
        ┌────────────────┐              │
        │  CALCULATE      │──── S105     │
        │ CORRESPONDENCE  │              │
        │ MATRIX          │              │
        └───────┬────────┘              │
                │                        │
        ┌────────────────┐              │
        │  COORDINATE     │──── S106     │
        │ TRANSFORMATION  │              │
        └───────┬────────┘              │
                │                        │
           ◇─────────────◇  S107         │
          │ IS MATCHING   │   No         │
          │  CORRECT?     │────┐    S109  │
           ◇─────────────◇    │   ┌──────────┐
             Yes│   S108       └──►│ CORRECT  │
        ┌────────────────┐        │ MATCHING │
        │  CALCULATE      │        └────┬─────┘
        │  SCORE          │          S110│
        └───────┬────────┘        ┌──────────┐
                │                  │   SET    │
        ┌────────────────┐        │CONSTRAINT│
        │     END         │        └────┬─────┘
        └────────────────┘             │
                                        └──────────┘
```

**EP 4 715 736 A1**

**Description**

This application relates to an image processing device, an image processing method, and a program.

Devices that perform position alignment for a comparison between medical images have been known (for example, Patent Literature 1 (Japanese Patent No. 5159301)). A medical image display device described in Patent Literature 1, when a user specifies several points of interest in two three-dimensional medical images, determines coordinate transformation parameters, based on position information of matched points and displays matching images.

One aspect of an image processing device (1) according to the present disclosure includes one or more processors (100) that acquire a first image and a second image each of which includes a plurality of partial image areas, perform matching between a plurality of the partial image areas included in the first image and a plurality of the partial image areas included in the second image, and accept correction by a user with respect to the matching between the partial image area included in the first image and the partial image area included in the second image. The one or more processors (100), using a matching associated with correction by the user as a constraint condition, perform matching between a plurality of the partial image areas other than the partial image area associated with the constraint condition in the first image and a plurality of the partial image areas other than the partial image area associated with the constraint condition in the second image again.

According to the present disclosure, it becomes possible to perform matching of comparison target images with higher accuracy.

A more complete understanding of this application can be obtained when the following detailed description is considered in conjunction with the following drawings, in which:

FIG. 1 is a block diagram illustrating an image processing device according to an embodiment of the present disclosure;
FIG. 2 is a diagram illustrating a display example of a detection screen of the image processing device;
FIG. 3 is a diagram illustrating a display example of a portion of the detection screen;
FIG. 4 is a diagram illustrating a display example of a matching screen of the image processing device;
FIG. 5 is a diagram illustrating a display example of a portion of the matching screen;
FIG. 6 is a diagram illustrating another display example of the portion of the matching screen;
FIG. 7 is a diagram illustrating another display example of the matching screen of the image processing device;
FIG. 8 is a diagram illustrating a display example of a score calculation screen of the image processing device; and
FIG. 9 is a flowchart of image processing.

An embodiment of the present disclosure is described below with reference to the drawings. Note that, in the drawings, the same or equivalent constituent elements are designated by the same reference numerals.

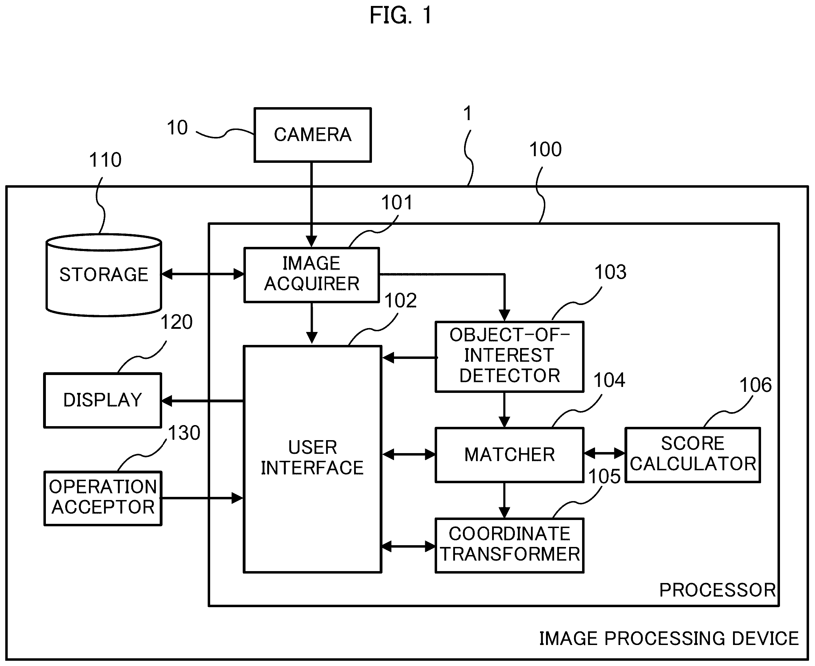

An image processing device 1 according to the embodiment of the present disclosure is a device that causes two images including a plurality of partial image areas in each of which an object of interest appears to be displayed in a comparable manner. The image processing device 1 includes, as illustrated in FIG. 1, a processor 100 that controls display of an image captured by a camera 10, a storage 110 that stores various types of data including images, a display 120 that causes an image to be displayed, and an operation accepter 130 that accepts operation by a user.

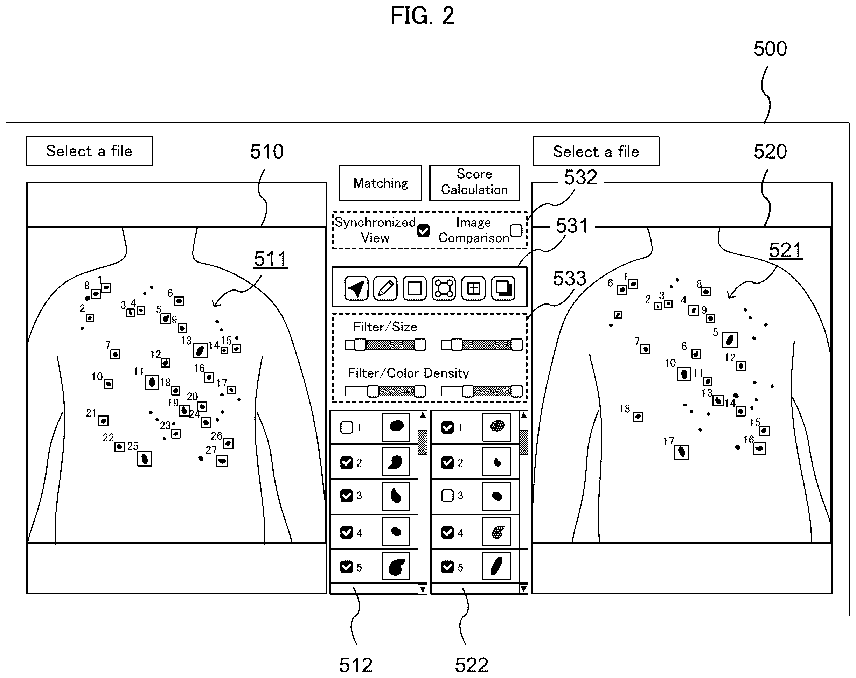

In this configuration, a first image and a second image that the image processing device 1 causes to be displayed are images in which the same subject is captured at timings different from each other, and in each of the images, a partial image area group that includes a plurality of partial image areas in each of which an object of interest with a chronological change has appeared is included. For example, the first image and the second image are medical images in which skin of a person or an animal is captured, and each partial image area is an area in which a candidate of a lesion on skin of the person or the animal has appeared. Lesions on the skin are, for example, pigment cell nevi (moles), melanomas, or the like. In a display example of the image processing device 1 in FIG. 2, a first medical image 510 (first image) on the left is a current image in which the back of a person is captured, and a second medical image 520 (second image) on the right is an image in which the back of the same person was captured in the past. More specifically, in the current first medical image 510, numerous skin lesion candidates that are objects of interest appear and there are a plurality of partial image areas each of which is enclosed by a square frame surrounding a lesion candidate, and a first partial image area group 511 including the partial image areas is included in the first medical image 510. In the past second medical image 520, numerous skin lesion candidates that are objects of interest also appear and there are a plurality of partial image areas each of which is enclosed by a square frame surrounding a lesion candidate, and a second partial image area group 521 including the partial image areas is included in the second medical image 520. By comparing partial image areas in the first medical image 510 and the second medical image 520 in detail, a chronological change in size, color, or a shape of each lesion candidate can be observed.

The camera 10 is, for example, a digital camera and transmits captured image data to the image processing device 1 by an arbitrary communication method. Although it is not required to exactly match an imaging range and orientation of the camera 10 between the first medical image 510 and the second medical image 520, the first medical image 510 and the second medical image 520 are preferably captured in approximately the same imaging range and orientation. Because of this configuration, a load of processing, such as matching partial image areas with each other, can be reduced.

The processor 100 of the image processing device 1 includes, for example, a central processing unit (CPU) and peripheral circuits of the CPU, and executes various types of operation processing. The processor 100 may include a single CPU, or may include a plurality of CPUs. The processor 100 executes programs for various types of operation processing, including image processing, that are stored in storage 110. The processor 100 may include volatile semiconductor memory, such as a random access memory (RAM), that functions as a working memory of the CPU. In addition, the processor 100 may further include an operation circuit, such as a logical operation unit and a numerical operation unit.

The storage 110 includes a non-volatile semiconductor memory, such as an electrically erasable and programmable read only memory (EEPROM) and a flash memory. The storage 110 stores medical images captured by the camera 10, programs for various types of operation processing, including image processing, that the processor 100 executes, and various types of data used for operation processing of the processor 100.

The display 120 is an arbitrary display device that displays medical images and an operation screen sent from the image processing device 1, and is, for example, a liquid crystal display or an organic electro-luminescence (EL) display. The display 120 displays a detection screen 500 that includes, as illustrated in FIG. 2, two image frames for displaying the first medical image 510 and the second medical image 520 that are arranged on the right and left sides and user interface elements (hereinafter, referred to as UI elements), such as operation buttons and operation bars, that are arranged around the first medical image 510 and the second medical image 520. The operation accepter 130 includes a pointing device, such as a mouse, a touch pad, and a touch panel, detects an operation on the first medical image 510, the second medical image 520, and the UI elements that are displayed on the display 120, and sends an operation signal to the processor 100. The display 120 and the operation accepter 130 may be an integrated touch panel display or the like.

By executing a program for image processing stored in the storage 110, the processor 100 functions as an image acquirer 101, a user interface 102, an object-of-interest detector 103, a matcher 104, a coordinate transformer 105, and a score calculator 106. The image acquirer 101 acquires a medical image captured by the camera 10, causes the acquired medical image to be stored in storage 110, and causes the medical image to be displayed on the display 120 via the user interface 102. The user interface 102 causes two image frames for causing the two images acquired by the image acquirer 101, that is, the first medical image 510 and the second medical image 520, to be displayed that are arranged on the right and left sides and the UI elements, such as operation buttons and operation bars, that are arranged around the image frames to be displayed. The user interface 102 acquires operation input performed by the operation accepter 130 on the first medical image 510, the second medical image 520, and the UI elements, and outputs the operation input to the matcher 104 and the coordinate transformer 105.

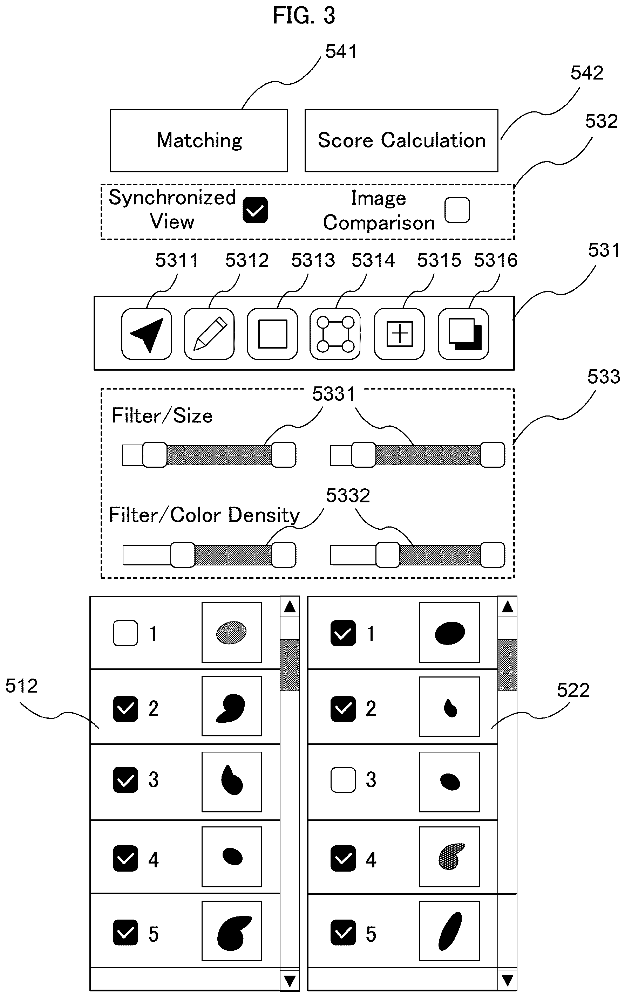

The object-of-interest detector 103 searches each of the first medical image 510 and the second medical image 520 for objects of interest and detects objects of interest, as illustrated in FIG. 2, and acquires central coordinates of each object of interest. As a detection method for an object of interest, an arbitrary method can be used, and, for example, a method using binarization processing and connected component analysis can be used. Alternatively, an object of interest may be detected by machine learning. On this occasion, the object-of-interest detector 103 may acquire size or a value of color density of an object of interest. As the size of an object of interest, for example, diameter of a perfect circle equivalent to area of a lesion candidate range where a lesion candidate appears, diameter of a circumcircle of the lesion candidate range, or a maximum diameter of the lesion may be acquired. In addition, as the color density, for example, a luminance difference between inside and outside a lesion candidate range may be acquired. The object-of-interest detector 103 causes an ID number serving as an identification number and central coordinates of a detected object of interest to be stored in the storage 110. In the example in FIG. 2, an object of interest that the object-of-interest detector 103 detects is a lesion candidate area that has appeared on human skin, and the object-of-interest detector 103 generates a square frame surrounding each object of interest and causes the generated frame to be displayed, and assigns an ID number to each object of interest. An area enclosed by a square frame is a partial image area, and a plurality of partial image areas is displayed in the first medical image 510 and the second medical image 520 in an identifiable manner by ID numbers. Partial image areas included in the first partial image area group 511 in the first medical image 510 are enlarged and displayed vertically in a first partial image display area 512, and partial image areas included in the second partial image area group 521 in the second medical image 520 are enlarged and displayed vertically in a second partial image display area 522. As illustrated in FIG. 2 and FIG. 3 that is an enlarged view of a central portion of FIG. 2, a check box is provided beside the ID number of each of partial image areas in the first partial image display area 512 and the second partial image display area 522 and is used for selection of a partial image area that is to be used for matching. A partial image area that is selected using a check box is highlighted in the first medical image 510 and the second medical image 520. For example, color of the frame of a partial image area selected using a check box may be set to a different color from the colors of the frames of partial image areas not selected using check boxes.

The UI elements displayed on the display 120 include, as illustrated in FIG. 3, operation buttons 531 for switching operations on the first medical image 510, the second medical image 520, or partial image areas included in the foregoing. The operation buttons 531 are used to switch functions of the pointing device of the operation accepter 130. For example, when a button 5311 is selected, movement, enlargement, or reduction of the first medical image 510 or the second medical image 520 can be executed through operation of the pointing device. In addition, when a button 5312 is selected, an object of interest can be selected by enclosing the object of interest with a free curve. In addition, when a button 5313 is selected, an object of interest can be selected by enclosing the object of interest with a rectangle. In addition, when a button 5314 is selected, a partial image area can be edited by changing size of a frame enclosing an object of interest. In addition, when a button 5315 is selected, a partial image area can be newly created by drawing a frame. In addition, when a button 5316 is selected, a correction mode to correct matching between partial image areas in the first medical image 510 and the second medical image 520, which is described later, can be turned to an ON state.

In addition, the UI elements displayed on the display 120 include check boxes 532 for performing operation to synchronize the first medical image 510 and the second medical image 520 with each other or operation to switch an image to be displayed in each of the right and left image frames. Specifically, when a check box labeled "Synchronized View" is checked, the first medical image 510 and second medical image 520 displayed on the right and left sides are operated in synchronization with each other. For example, when the check box labeled "Synchronized View" is checked and the button 5311 is selected, the same movement, enlargement, or reduction can be simultaneously executed for both the first medical image 510 and the second medical image 520. When a check box labeled "Image Comparison" is checked, the image frames in which the first medical image 510 and the second medical image 520 are displayed can be exchanged between the right and left image frames. More specifically, it is possible to display the second medical image 520 in the left image frame while leaving frames indicating partial image areas created for the first medical image 510 in the left image frame. After the coordinate transformation processing, which is described later, is performed, an image after coordinate transformation of the second medical image 520 can be displayed when the second medical image 520 is displayed in the left image frame. Because of this configuration, it is possible to visually recognize changes in the objects of interest between the first medical image 510 and the second medical image 520.

In addition, as another UI element, a filter setter 533 is displayed in the display screen. The filter setter 533 is a UI element that is indicators to specify a range of at least one of the size and color density of an object of interest, using range sliders 5331 or 5332 and that achieves a filtering function to select an object of interest within the specified range. Lower limits of size and color density can be set with left knobs of the range sliders 5331 and 5332, respectively, and upper limits of size and color density can be set with right knobs of the range sliders 5331 and 5332, respectively. It may be configured such that when the knobs are positioned at the left ends or the right ends of the range sliders 5331 or 5332, filtering by the lower limit or the upper limit of size or color density is not performed. For example, it may be configured such that as illustrated in FIG. 3, when a range of size of a lesion candidate area serving as an object of interest is set using the range sliders 5331, a partial image area of a lesion candidate having a size within the set range among the first partial image area group 511 and the second partial image area group 521 is selected and a check box corresponding to the partial image area is checked. In addition, it may also be configured such that when a range of color density of a lesion candidate area serving as an object of interest is set using the range sliders 5332, a partial image area of a lesion candidate having a color density within the set range among the first partial image area group 511 and the second partial image area group 521 is selected and a check box corresponding to the partial image area is checked. The size of an object of interest may be represented by diameter of a perfect circle equivalent to area of the lesion candidate area, diameter of a circumcircle of the lesion candidate area, or a maximum diameter of the lesion candidate area. In addition, the color density may be represented by, for example, a luminance difference between inside and outside the lesion candidate range. A partial image area selected using a check box is highlighted in the first medical image 510 and the second medical image 520. Alternatively, a partial image area selected using a check box is used as a target of matching performed by the matcher 104. When the check box 532 labeled "Synchronized View" is checked and the first medical image 510 and the second medical image 520 are synchronized with each other, the range sliders in the filter setter 533 are used in a synchronized manner between the first partial image area group 511 and the second partial image area group 521 and the same range from a lower limit to an upper limit may be selected for both the first partial image area group 511 and the second partial image area group 521.

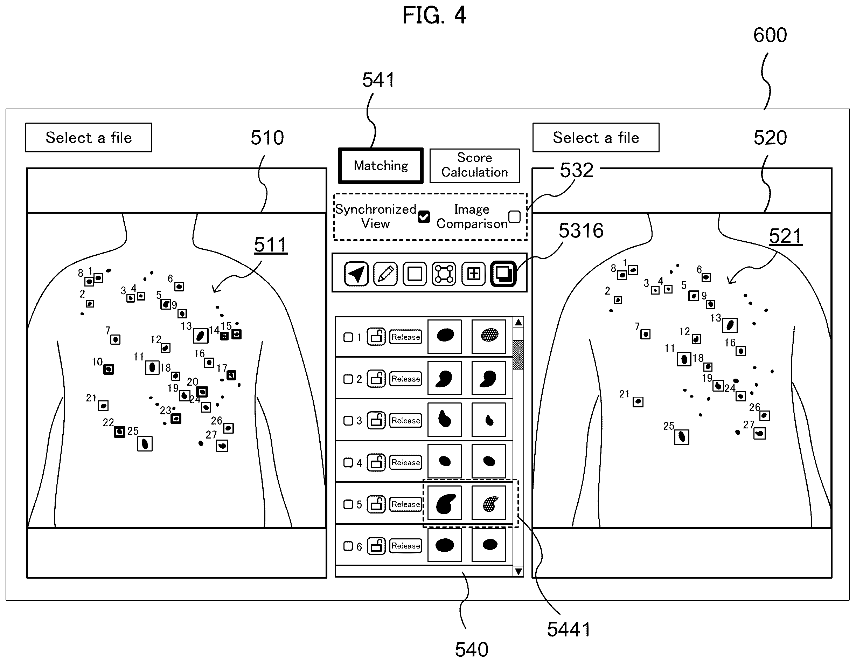

The matcher 104 performs matching of partial image areas of objects of interest that are selected by the user or selected through the filter setting among objects of interest detected by the object-of-interest detector 103 from the first medical image 510 and the second medical image 520. When a button 541 for matching is selected as illustrated in FIG. 4, the matching by the matcher 104 is executed. The matching is performed using an arbitrary algorithm. For example, a thin plate spline robust point matching (TPS-RPM) algorithm is used. In the TPS-RPM algorithm, an objective function expressed by the equation (1) below is calculated. In the equation (1), $x_i$ denotes coordinates of the center point of an i-th object of interest in the first medical image 510, $y_j$ denotes coordinates of the center point of a j-th object of interest in the second medical image 520, the number of objects of interest in the first medical image 510 is denoted by N, the number of objects of interest in the second medical image 520 is denoted by L, f is a mapping function, and P is a correspondence matrix. The second term in the equation (1) is a term used for adjustment of bending energy and is expressed by the equation (2).
[Math. 1]

$$E(\boldsymbol{P}, f) = \sum_{j=1}^{N} \sum_{i=1}^{L} p_{ij} \left\| \boldsymbol{y}_j - f(\boldsymbol{x}_i) \right\|^2 + \lambda \|\mathcal{L}f\|^2 - \zeta \sum_{j=1}^{N} \sum_{i=1}^{L} p_{ij} \qquad \cdots (1)$$

$$\begin{pmatrix} p_{ij} \in \{0,1\}, & for \ i = 1,2,\cdots,L; j = 1,2,\cdots,N \\ \sum_{j=1}^{N+1} p_{ij} = 1 & for \ i = 1,2,\cdots,L \\ \sum_{j=1}^{L+1} p_{ij} = 1 & for \ i = 1,2,\cdots,N \end{pmatrix}$$

[Math. 2]

$$\|\mathcal{L}f\|^2 = \iint \left[ \left(\frac{\partial^2 f}{\partial u^2}\right)^2 + 2\left(\frac{\partial^2 f}{\partial u \partial v}\right)^2 + \left(\frac{\partial^2 f}{\partial v^2}\right)^2 \right] du dv \qquad \cdots (2)$$

The matcher 104 calculates a correspondence matrix P that minimizes the objective function E expressed by the equation (1) and determines objects of interest corresponding to each other. The correspondence matrix P is a matrix with (L+1) rows and (N+1) columns, where 1 is added to the number of objects of interest to represent lack of matching. As a result of matching by the matcher 104, a list 540 obtained by displaying in an enlarged manner and arranging in parallel pieces of matching result information 5441 each of which combines a partial image area in the first medical image 510 and a partial image area in the second medical image 520, as illustrated in FIG. 4 is displayed in a matching screen 600. On this occasion, the ID numbers are reassigned in, for example, ascending order.

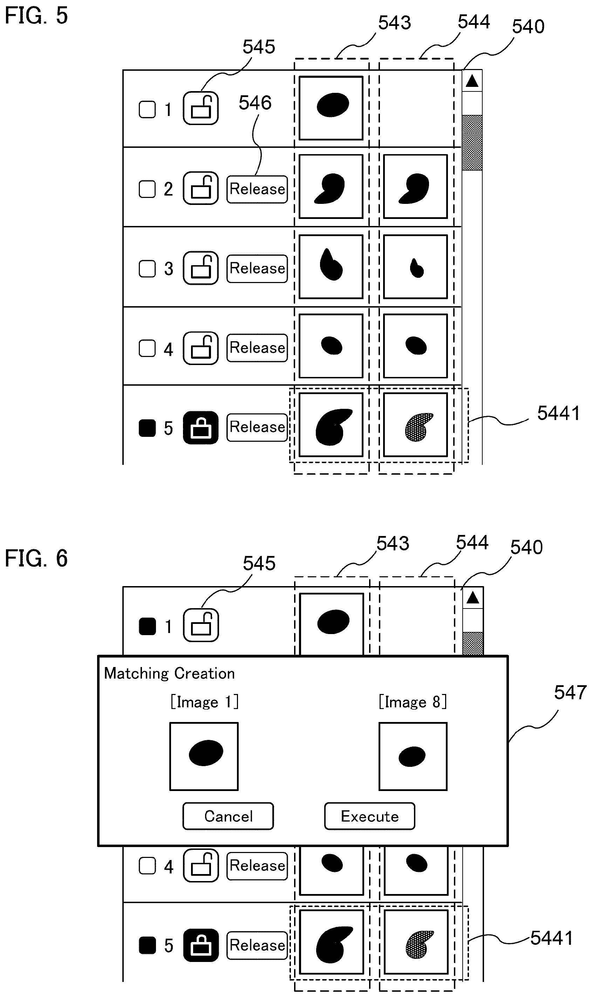

For the result of matching by the matcher 104, the user can set a constraint condition and make a correction. Using FIG. 5 in which the list 540 in the matching screen 600 illustrated in FIG. 4 is illustrated in an enlarged manner, a procedure of operation performed by the user is described in detail. As illustrated in FIG. 5, in the matching screen 600, pieces of matching result information 5441 each of which indicates a combination of a partial image area 543 in the first medical image 510 and a partial image area 544 in the second medical image 520 that are matched with each other and lock buttons 545 (fixing operation elements) and release buttons 546 (release operation element) that are arranged alongside the pieces of matching result information 5441 are displayed. The user can fix one of the combinations of a partial image area 543 and a partial image area 544, as a constraint condition when performing matching processing to be performed in the following step. FIG. 5 illustrates a state in which by turning a lock button 545 of a combination with ID number 5 to the ON state, the combination is fixed. The matcher 104 performs reprocessing of matching with respect to a plurality of partial image areas 543 other than the partial image area 543 associated with the constraint condition and a plurality of partial image areas 544 other than the partial image area 544 associated with the constraint condition.

In addition, the user can correct each of combinations of a partial image area 543 in the first partial image area group 511 and a partial image area 544 in the second partial image area group 521 that are matched with each other by the matcher 104. FIG. 5 illustrates a state in which by selecting a release button 546 of a combination with ID number 1, matching is released. Subsequently, the user, after selecting the button 5316 illustrated in FIG. 4, selects a partial image area 544 that is considered to correctly match the partial image area 543 in the first medical image 510 the matching of which has been released, from the second medical image 520. In the example in FIG. 4, although a partial image area 543 with ID number 1 in the first medical image 510 is matched with a partial image area 544 with ID number 1 in the second medical image 520, it is considered that the partial image area 543 with the ID number 1 in the first medical image 510 should be correctly matched with a partial image area 544 with ID number 8 in the second medical image 520. In that case, the user can select the partial image area with the ID number of 8 from the second medical image 520. On this occasion, since a confirmation screen 547 as illustrated in FIG. 6 is displayed, the user can correct the matching by selecting an execution option in this confirmation screen 547. When a release operation is performed in this way, correction operation of newly matching, with a partial image area included in one of the first partial image area group 511 and the second partial image area group 521 that is associated with the release operation, a partial image area included in the other of the first partial image area group 511 and the second partial image area group 521 can be performed. Note that as for a combination the matching of which is corrected by the user, the combination may be used as a constraint condition by turning the lock button 545 of the combination to the ON state. Because of this configuration, the matcher 104 is capable of performing reprocessing of matching with respect to a plurality of partial image areas 543 other than the partial image area 543 associated with the correction by the user and a plurality of partial image areas 544 other than the partial image area 544 associated with the correction by the user.

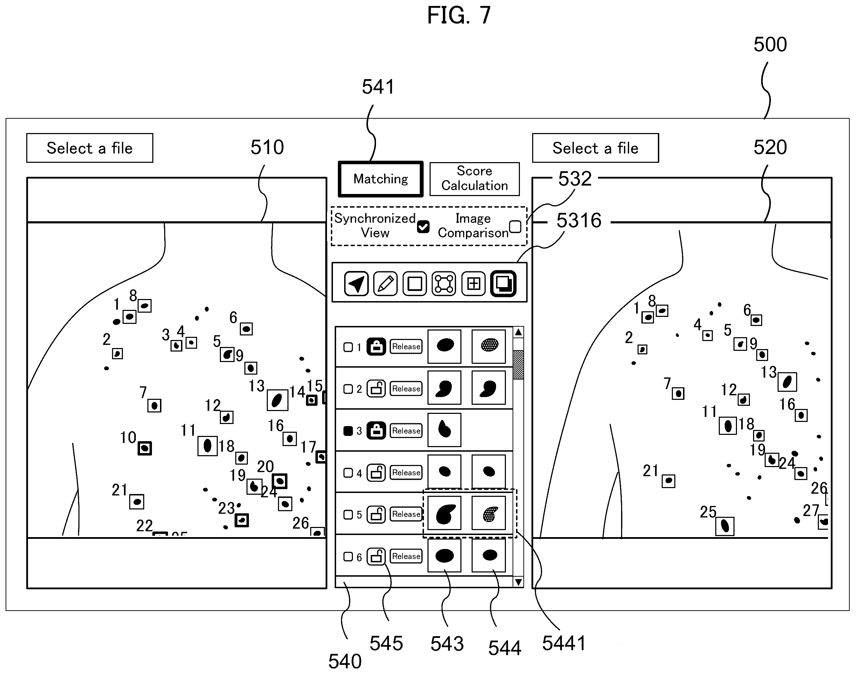

In addition, there is a case where no matching is established since no partial image area matching a partial image area included in one of the first partial image area group 511 in the first medical image 510 and the second partial image area group 521 in the second medical image 520 is included in the other. For example, matching is not established in a case where a skin lesion candidate area newly emerges or disappears. In that case, it is also possible to set the lack of matching as a constraint condition. For example, as illustrated in FIG. 7, no object of interest matching an object of interest in a partial image area 543 with ID number 3 in the first medical image 510 does not exist in the second medical image 520. In this case, as illustrated in the list 540 in FIG. 7, a partial image area 544 with the ID number 3 is left blank, and a lock button 545 of the partial image area 544 is turned to the ON state. Through this operation, it is possible to fix the lack of matching for the partial image area 543 with ID number 3 and perform matching processing with respect to partial image areas 543 with ID numbers other than 3.

The coordinate transformer 105 performs a nonlinear coordinate transformation indicated by the correspondence matrix calculated by the matcher 104 on one of the first medical image 510 and the second medical image 520 and thereby corrects the image. For example, an image to which the second medical image 520 is corrected by performing the nonlinear coordinate transformation on the second medical image 520 may be displayed in the right image frame. Alternatively, when by turning the button labeled "Image Comparison" in the display screen in FIG. 4 to the ON state, the image frames of the first medical image 510 and the second medical image 520 are exchanged between the right and left image frames and the second medical image 520 is displayed in the left image frame, the second medical image 520 on which the nonlinear coordinate transformation is performed is displayed. On this occasion, since the second medical image 520 after nonlinear coordinate transformation is displayed in the left image frame while leaving the frames of the partial image areas in the first medical image 510 in the left image frame, it becomes easier to visually recognize chronological changes between corresponding lesion candidate areas.

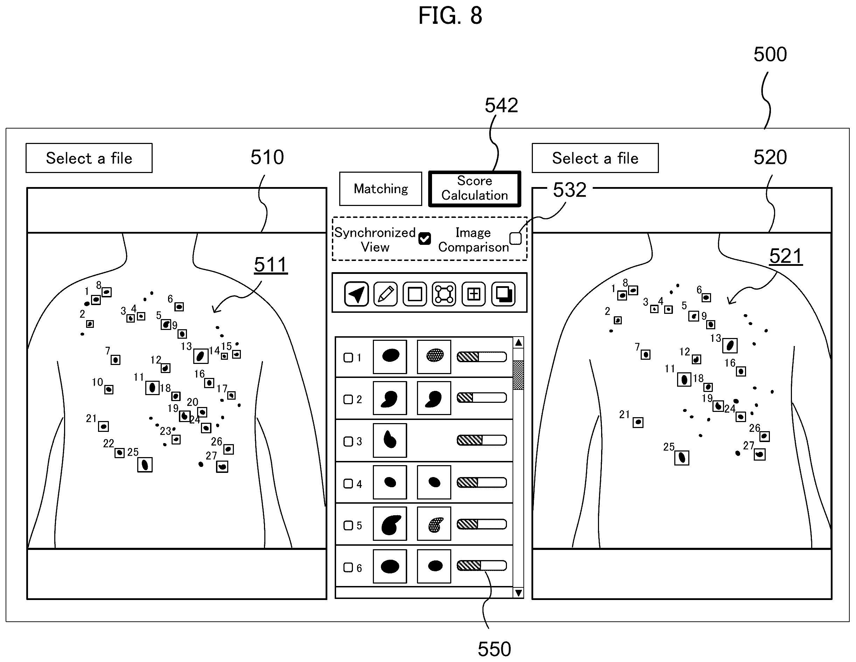

The matcher 104 can bring an accuracy rate in matching close to 100% by repeatedly executing the matching processing after setting of the constraint condition and correction of the matching performed by the user. After the matching performed by matcher 104, the score calculator 106 performs a comparison between the partial image areas in the first medical image 510 and the partial image areas in the second medical image 520 that are matched with each other, and calculates a score indicating a degree of change. Although as a method for calculating the score, an arbitrary method can be used, in the case of skin lesion candidate areas, the calculation may be performed based on a rate of change in the colors and sizes of the lesion candidate areas. The score calculation is executed when a button 542 for score calculation is selected, as illustrated in FIG. 8. A score calculation result may be represented by score data bars 550 or the like, as illustrated in FIG. 8.

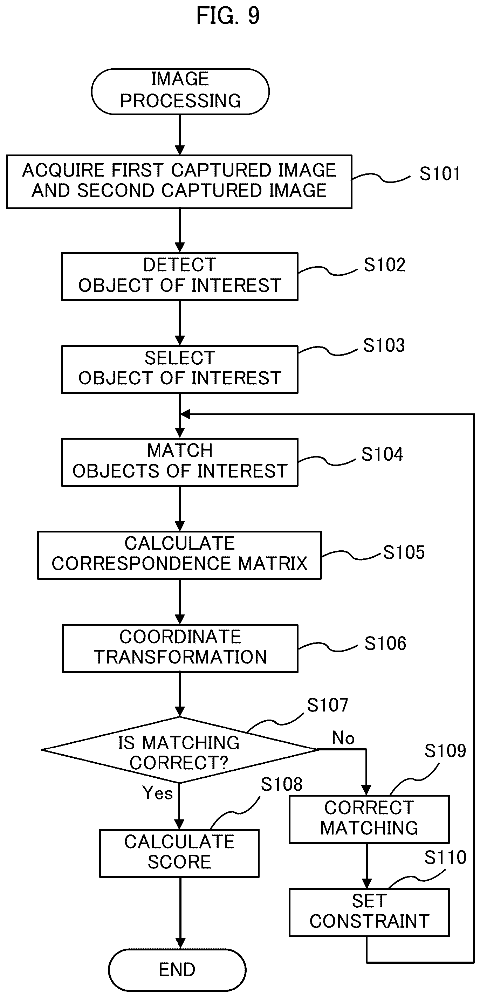

Operation of the image processing device 1 described above is described in accordance with a flowchart in FIG. 9. First, by the user selecting files of captured images that the user desires to evaluate with the image processing device 1, the image acquirer 101 acquires a first medical image 510 and a second medical image 520 (step S101), and causes the first medical image 510 to be displayed in the left image frame in the detection screen 500 and causes the second medical image 520 to be displayed in the right image frame in the detection screen 500, as illustrated in FIG. 2. Next, the object-of-interest detector 103 detects objects of interest from each of the first medical image 510 and the second medical image 520 (step S102). For example, when the first medical image 510 and the second medical image 520 are images of the back of a person as illustrated in FIG. 2, the object-of-interest detector 103 detects lesion candidate areas having emerged on the skin. The object-of-interest detector 103 acquires central coordinates of each of the detected plurality of objects of interest, assigns an ID number serving as an identification number to each object of interest, and stores the ID numbers, the central coordinates, and the partial image areas where the objects of interest appear, in the storage 110. In addition, the object-of-interest detector 103 generates a square frame enclosing each object of interest and causes the generated frames to be displayed in the detection screen 500, and assigns an ID number to a partial image area of each object of interest. In addition, the object-of-interest detector 103 displays partial image areas included in the first partial image area group 511 in the first medical image 510 vertically in the first partial image display area 512 in an enlarged manner, and displays partial image areas included in the second partial image area group 521 in the second medical image 520 vertically in the second partial image display area 522 in an enlarged manner.

The matcher 104 selects objects of interest to be matched from among the objects of interest in the first medical image 510 and the second medical image 520 that are detected by the object-of-interest detector 103 (step S103). The selection of objects of interest is performed based on selection operation by the user on check boxes provided beside the ID numbers of the partial image areas in the first partial image display area 512 and the second partial image display area 522 illustrated in FIG. 2. Alternatively, when a range of at least one of the size and color density of an object of interest is specified by the user operating the range sliders 5331 and 5332 of the filter setter 533 in the detection screen 500, an object of interest within the specified range is selected as a target of the matching processing. The check box provided beside a partial image area in which the selected object of interest appears is checked.

The matcher 104 matches the partial image areas of objects of interest that are selected by the user or selected through the filter setting from among the objects of interest detected by the object-of-interest detector 103 from the first medical image 510 and the second medical image 520 (step S104). When the button 541 for matching is selected as illustrated in FIG. 4, the matching by the matcher 104 is executed. As a result of the matching by the matcher 104, a correspondence matrix that indicates correspondence relationships between the central coordinates of the objects of interest in the first medical image 510 and the central coordinates of the objects of interest in the second medical image 520 is calculated (step S105). The coordinate transformer 105 performs a coordinate transformation on one of the first medical image 510 and the second medical image 520, based on the correspondence matrix calculated in step S105 (step S106), and displays an image after coordinate transformation in the image frame on one of the right and left sides.

A plurality of pairs of partial image areas in the first medical image 510 and the second medical image 520 that are matched by the matcher 104 is displayed in the list 540 illustrated in FIG. 4. The user checks the first medical image 510, the second medical image 520, and the list 540 of pairs of matching partial image areas and confirms whether or not there is any incorrect matching. When the matching is determined to be completely correct by the user (step S107: Yes) and the button 542 for score calculation is selected by the user, the score calculator 106 calculates a score indicating a chronological change for each partial image area (step S108). Subsequently, the process terminates. In the case where objects of interest are skin lesion candidate areas, the score may be calculated based on rates of change in the colors and sizes of the lesion candidate areas.

When the matching is determined to be incorrect (step S107: No), the user performs an operation to correct the matching (step S109). Specifically, by selecting a release button 546 as illustrated in FIG. 5 arranged alongside a partial image area 543 in the first medical image 510 and a partial image area 544 in the second medical image 520 that are matched with each other, the matching between the partial image area 543 and the partial image area 544 is released. By selection operation by the user on the first medical image 510 and the second medical image 520, a new matching is generated. When a new matching is generated by the correction performed by the user, a constraint condition to fix the matching is set (step S110). Subsequently, the process returns to step S104, and the matcher 104 performs matching with respect to partial image areas 543 and 544 other than the partial image areas 543 and 544 associated with the constraint condition (step S104). Subsequently, the matcher 104 calculates the correspondence matrix indicating correspondence relationships between the central coordinates of the objects of interest in the first medical image 510 and the central coordinates of the objects of interest in the second medical image 520 again (step S105), and the coordinate transformer 105 performs the coordinate transformation on one of the first medical image 510 and the second medical image 520 (step S106) and causes an image after coordinate transformation to be displayed in the image frame on one of the right and left sides. When the processing from steps S104 to S106 is repeated and the user determines the matching to be completely correct, as described above (step S107: Yes), by the button 542 for score calculation being selected, the score calculator 106 calculates a score indicating a chronological change with respect to each partial image area (step S108), and the process terminates.

As described in the foregoing, in the image processing device 1 according to the present embodiment, the image acquirer 101 acquires the first medical image 510 including the first partial image area group 511 and the second medical image 520 including the second partial image area group 521, the object-of-interest detector 103 detects an object of interest from each of the first medical image 510 and the second medical image 520, and the matcher performs matching of the partial image areas in the first medical image 510 and the second medical image 520 in which the objects of interest appear. The matcher 104 is configured to accept correction of matching by the user with respect to matching of a plurality of pairs of partial image areas and, using a matching associated with the correction by the user as a constraint condition, perform matching between a plurality of partial image areas other than a partial image area associated with the constraint condition in the first medical image 510 and a plurality of partial image areas other than a partial image area associated with the constraint condition in the second medical image 520 again. Because of this configuration, it becomes possible to accurately perform a comparison between matching partial image areas.

In addition, the object-of-interest detector 103 is configured to acquire the size or color density value of each of objects of interest appearing in partial image areas and, when the user performs an operation to specify a range of at least one of the size and color density of an object of interest on the detection screen 500, display the partial image area of an object of interest matching the range of size or color density specified by the user operation in a highlighted manner in each of the partial image areas in the first medical image 510 and the partial image areas in the second medical image 520. Because of this configuration, it becomes possible to easily select an object of interest.

In addition, the user performing an operation to specify a range of at least one of the size or color density of an object of interest on the detection screen 500 enables the image processing device 1 to easily narrow down matching results between objects of interest.

In addition, the matcher 104 displays on the display 120 the matching screen 600 that includes the first medical image 510 and the second medical image 520 in which matching of a plurality of pairs of partial image areas is performed and the list 540 in which a plurality of pairs of a partial image area included in the first partial image area group 511 and a partial image area included in the second partial image area group 521 that are matched with each other is displayed in an enlarged manner and is arranged in parallel, and accepts correction by the user with respect to the matching between the partial image areas included in the first partial image area group and the partial image areas included in the second partial image area group. It is configured such that the list 540 includes a release operation element that releases each of matchings of a plurality of pairs of partial image areas and when a release operation is performed on the release operation element, the matcher 104 accepts an operation by the user to newly match, with a partial image area included in one of the first partial image area group 511 and the second partial image area group 521 that is associated the release operation, a partial image area included in the other. Because of this configuration, it becomes possible to improve an accuracy rate of matching of partial image areas.

Although in the prior art, when orientations, sizes, and like of comparison target images are differ from each other, it is difficult to perform matching with high precision, the present disclosure enables matching of comparison target images to be performed with higher accuracy.

Although the embodiment of the present disclosure is described above, the above-described embodiment is only an example, and the scope of application of the present disclosure is not limited to the embodiment. That is, various applications of the embodiment of the present disclosure are possible, and all embodiments are included in the scope of the present disclosure.

For example, although in the embodiment, the matcher 104 is configured to perform matching with respect to objects of interest detected by the object-of-interest detector 103, it may be configured such that some patterns of matching based on user selection are performed before the matching by the matcher 104 and with fixation of the matchings used as a constraint condition, the matching by the matcher 104 is performed. Because of this configuration, a load due to processing of matching by the matcher 104 can be reduced, and the accuracy rate can also be improved.

In addition, although in the embodiment, it is configured such that two medical images can be displayed and compared, three or more medical images may be displayed. In addition, out of three or more medical images, two images may be selected and matched.

In addition, although in the embodiment, it is configured such that using the first medical image 510 and the second medical image 520 in which human skin is captured as the first image and the second image, respectively, lesion candidate areas that have emerged on the skin are detected and matched as objects of interest, the present disclosure is not limited the configuration. The first image and the second image may be another type of medical images, and may be, for example, endoscope images or tomographic images. Alternatively, images other than medical images may be used, and, for example, images of an outer wall of a building or the like may be used. In that case, deterioration, damage, or the like having occurred on the outer wall may be detected as an object of interest and matching of such objects of interest may be performed.

In addition, although in the above-described embodiment, the image processing program executed by the processor 100 is stored in advance in the nonvolatile memory of the storage 110, the present disclosure is not limited thereto. By implementing a program to cause the above-described various types of processing to be executed on an existing general-purpose computer or the like, the computer may be caused to function as an image processing device according to the embodiment described above.

A method for distributing such a program is arbitrarily determined, and, for example, the program may be distributed stored in a non-transitory computer-readable recording medium (such as a flexible disk, a compact disk (CD)-ROM, a digital versatile disk (DVD)-ROM, a magneto optical disk (MO), a memory card, and a USB memory) or may be stored in a storage on a network, such as the Internet, and provided by having the program downloaded from the storage via the network.

When the above-described processing is to be achieved through sharing between an operating system (OS) and an application program or collaboration between the OS and the application program, only the application program may be stored in a non-transitory recording medium or a storage device. It is also possible to superimpose a program on a carrier wave and distribute the program via a network. For example, the above-described program may be posted on a bulletin board system (BBS) on the network, and the program may be distributed via the network. The above-described processing may be configured to be able to be performed by starting up and executing the distributed program in a similar manner to other application programs under the control of the OS.

In addition, the processor 100 may be configured not only by an arbitrary processor, such as a single processor, multiple processors, and a multi-core processor, alone but also by combining such an arbitrary processor and a processing circuit, such as an application specific integrated circuit (ASIC) and a field-programmable gate array (FPGA).

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

**Claims**

1. An image processing device (1), comprising
   one or more processors (100) configured to:

   acquire a first image and a second image each of which includes a plurality of partial image areas and perform matching between a plurality of the partial image areas included in the first image and a plurality of the partial image areas included in the second image; and
   accept correction by a user with respect to the matching between the partial image area included in the first image and the partial image area included in the second image,
   wherein the one or more processors (100), using a matching associated with correction by the user as a constraint condition, perform matching between a plurality of the partial image areas other than the partial image area associated with the constraint condition in the first image and a plurality of the partial image areas other than the partial image area associated with the constraint condition in the second image again.

2. The image processing device (1) according to claim 1,

   wherein each of a plurality of the partial image areas included in the first image and the second image is an image area in which one object of interest appears, and
   the one or more processors (100) detect the object of interest from the first image and the second image and perform matching of the partial image areas in which the detected object of interest appears, and
   cause the first image and the second image in which matching of a plurality of pairs of the partial image areas is performed to be displayed on a display (120).

3. The image processing device (1) according to claim 2, wherein the one or more processors (100) calculate a correspondence matrix that matches central coordinates of the objects of interest included in the partial image areas in the first image and the second image with each other and perform correction based on a nonlinear coordinate transformation indicated by the correspondence matrix on one of the first image and the second image.

4. The image processing device (1) according to any one of claims 1 to 3,

   wherein in a case where the partial image area matching the partial image area included in one of the first image and the second image is not included in the other and matching is not established, the one or more processors (100) accept correction by the user indicating lack of matching, and
   the one or more processors (100), using lack of matching associated with correction by the user as a constraint condition, perform matching again with respect to a plurality of the partial image areas other than the partial image area associated with the constraint condition in one of the first image and the second image.

5. The image processing device (1) according to any one of claims 1 to 4, wherein the one or more processors (100) further accept selection by the user of the partial image area to be matched among a plurality of the partial image areas included in each of the first image and second image.

6. The image processing device (1) according to any one of claims 1 to 5, wherein the first image and the second image are medical images.

7. The image processing device (1) according to claim 6, wherein the first image and the second image are the medical images in which skin of a person or an animal is captured, and the first image and the second image include, as the object of interest, the partial image area in which a lesion candidate on skin of the person or the animal appears.

8. An image processing method, comprising

   acquiring, by one or more processors (100), a first image and a second image each of which includes a plurality of partial image areas;
   performing, by the one or more processors (100), matching between a plurality of the partial image areas included in the first image and a plurality of the partial image areas included in the second image;
   accepting, by the one or more processors (100), correction by a user with respect to the matching between the partial image area included in the first image and the partial image area included in the second image; and
   performing, by the one or more processors (100), using a matching associated with correction by the user as a constraint condition, matching between a plurality of the partial image areas other than the partial image area associated with the constraint condition in the first image and a plurality of the partial image areas other than the partial image area associated with the constraint condition in the second image again.

9. A program for executing processing comprising:

acquiring a first image and a second image each of which includes a plurality of partial image areas;
performing matching between a plurality of the partial image areas included in the first image and a plurality of the partial image areas included in the second image;
functioning as a user interface (102) that accepts correction by a user with respect to the matching between the partial image area included in the first image and the partial image area included in the second image; and
performing, using a matching associated with correction by the user as a constraint condition, matching between a plurality of the partial image areas other than the partial image area associated with the constraint condition in the first image and a plurality of the partial image areas other than the partial image area associated with the constraint condition in the second image again.

FIG. 1

CAMERA 10

IMAGE PROCESSING DEVICE 1

PROCESSOR 100

STORAGE 110

IMAGE ACQUIRER 101

OBJECT-OF-INTEREST DETECTOR 103

DISPLAY 120

USER INTERFACE 102

MATCHER 104

SCORE CALCULATOR 106

OPERATION ACCEPTOR 130

COORDINATE TRANSFORMER 105

EP 4 715 736 A1

# FIG. 2

Select a file    510

Select a file    520

500

511

521

Matching    Score Calculation    532

Synchronized View ☑   Image Comparison ☐   531

533

Filter/Size

Filter/Color Density

512    522

EP 4 715 736 A1

## FIG. 3

FIG. 4

FIG. 5

545 543 544 540

546

□ 1 🔓

□ 2 🔓 Release

□ 3 🔓 Release

□ 4 🔓 Release

■ 5 🔒 Release

5441

FIG. 6

545 543 544 540

■ 1 🔓

Matching Creation

[Image 1]  [Image 8]

Cancel  Execute

547

□ 4 🔓 Release

■ 5 🔒 Release

5441

15

FIG. 7

# FIG. 8

500

542

Select a file — 510

Matching | Score Calculation — 532

Synchronized View ☑ | Image Comparison ☐

511

Select a file — 520

521

550

EP 4 715 736 A1

## FIG. 9

IMAGE
PROCESSING

ACQUIRE FIRST CAPTURED IMAGE
AND SECOND CAPTURED IMAGE — S101

DETECT
OBJECT OF INTEREST — S102

SELECT
OBJECT OF INTEREST — S103

MATCH
OBJECTS OF INTEREST — S104

CALCULATE
CORRESPONDENCE MATRIX — S105

COORDINATE
TRANSFORMATION — S106

S107
IS MATCHING
CORRECT?

No

Yes

S108
CALCULATE
SCORE

S109
CORRECT
MATCHING

S110
SET
CONSTRAINT

END

# EUROPEAN SEARCH REPORT

**Application Number**

EP 25 19 9617

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 800 636 B1 (ALCON REFRACTIVE HORIZONS INC [US]) 24 September 2008 (2008-09-24) * the whole document * ----- | 1-9 | INV. G06T7/00 G06T7/33 |
| X | US 2020/265568 A1 (KOBAYASHI MITSUTOSHI [JP] ET AL) 20 August 2020 (2020-08-20) * paragraph [0056] - paragraph [0058] * * paragraph [0080] * * paragraph [0131] * * figure 3 * ----- | 1-9 | |
| A | DANIEL MARKEL ET AL: "A 4D biomechanical lung phantom for joint segmentation/registration evaluation", PHYSICS IN MEDICINE AND BIOLOGY, INSTITUTE OF PHYSICS PUBLISHING, BRISTOL GB, vol. 61, no. 19, 20 September 2016 (2016-09-20), pages 7012-7030, XP020309351, ISSN: 0031-9155, DOI: 10.1088/0031-9155/61/19/7012 [retrieved on 2016-09-20] * 2.4 * ----- | 1-9 | |

**TECHNICAL FIELDS SEARCHED (IPC)**

G06T

The present search report has been drawn up for all claims

| Place of search | Date of completion of the search | Examiner |
|---|---|---|
| Munich | 9 January 2026 | Gitzenis, Savvas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 19 9617

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2026

| Patent document cited in search report | | Publication date | Patent family member(s) | | Publication date |
|---|---|---|---|---|---|
| EP 1800636 | B1 | 24-09-2008 | AT | E409019 T1 | 15-10-2008 |
| | | | AU | 2006252201 A1 | 12-07-2007 |
| | | | BR | PI0605526 A | 16-10-2007 |
| | | | CA | 2570939 A1 | 22-06-2007 |
| | | | EP | 1800636 A1 | 27-06-2007 |
| | | | ES | 2316016 T3 | 01-04-2009 |
| | | | JP | 5006020 B2 | 22-08-2012 |
| | | | JP | 2007175494 A | 12-07-2007 |
| | | | KR | 20070066924 A | 27-06-2007 |
| | | | TW | I411429 B | 11-10-2013 |
| | | | US | 2007146633 A1 | 28-06-2007 |
| US 2020265568 | A1 | 20-08-2020 | CN | 111095351 A | 01-05-2020 |
| | | | DE | 112017007821 T5 | 16-04-2020 |
| | | | JP | 6790282 B2 | 25-11-2020 |
| | | | JP | WO2019053839 A1 | 28-05-2020 |
| | | | US | 2020265568 A1 | 20-08-2020 |
| | | | WO | 2019053839 A1 | 21-03-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5159301 B